# FASCICULE DE BREVET EUROPEEN

(11) **EP 1 885 673 B1**
(45) Date de publication et mention de la délivrance du brevet: **12.08.2015**
(21) Numéro de dépôt: 06755269.5
(22) Date de dépôt: 19.05.2006
(51) Int. Cl.: C07C 29/62, C07C 31/36, C07C 31/42

(54) **FABRICATION DE CHLORHYDRINE EN PHASE LIQUIDE EN PRESENCE DE COMPOSES LOURDS**
VERFAHREN ZUR HERSTELLUNG VON CHLORHYDRIN IN FLÜSSIGER PHASE IN GEGENWART VON SCHWEREN VERBINDUNGEN
METHOD FOR MAKING CHLOROHYDRIN IN LIQUID PHASE IN THE PRESENCE OF HEAVY COMPOUNDS

(30) Priorité: 20.05.2005 EP 05104321; 20.05.2005 FR 0505120; 08.11.2005 US 734635 P; 08.11.2005 US 734657 P; 08.11.2005 US 734636 P; 08.11.2005 US 734627 P; 08.11.2005 US 734634 P; 08.11.2005 US 734658 P; 08.11.2005 US 734637 P; 08.11.2005 US 734659 P
(43) Date de publication de la demande: 13.02.2008
(73) Titulaire: SOLVAY SA, 1120 Bruxelles (BE)
(72) Inventeur: GILBEAU, Patrick, B-7090 Braine-le-Comte (BE)
(74) Mandataire: Vande Gucht, Anne
(86) Numéro de dépôt international: PCT/EP2006/062445
(87) Numéro de publication internationale: WO 2006/100316

(56) Documents cités:
- WO-A-2005/021476
- WO-A-2005/054167
- US-A- 2 144 612
- GIBSON G P: "THE PREPARATION, PROPERTIES AND USES OF GLYCEROL DERIVATIVES. Part III. THE CHLOROHYDRINS" CHEMISTRY AND INDUSTRY, CHEMICAL SOCIETY, LECHWORTH, GB, 1931, pages 949-975, XP009042263 ISSN: 0009-3068

## Description

La présente invention se rapporte à un procédé de fabrication de dichloropropanol. Elle se rapporte plus spécifiquement à un procédé de fabrication de dichloropropanol par chloration de glycérol dans une phase liquide comprenant des composés lourds.

Le dichloropropanol est un intermédiaire réactionnel dans la fabrication de l'épichlorhydrine et des résines époxy (Kirk-Othmer Encyclopedia of Chemical Technology, Fourth Edition, 1992, Vol. 2, page 156, John Wiley & Sons, Inc.).

Selon des procédés connus, on peut obtenir le dichloropropanol notamment par hypochloration du chlorure d'allyle, par chloration de l'alcool allylique et par hydrochloration du glycérol. Ce dernier procédé présente l'avantage que le dichloropropanol peut être obtenu au départ de matières premières fossiles ou de matières premières renouvelables et il est connu que les ressources naturelles pétrochimiques, dont sont issues les matières fossiles, par exemple le pétrole, le gaz naturel ou le charbon, disponibles sur la terre sont limitées.

La demande internationale WO 2005/021476 décrit un procédé de fabrication de dichloropropanol par réaction entre du glycérol et du chlorure d'hydrogène gazeux en absence de solvant. Le dichloropropanol est séparé par des opérations de distillation successives et le résidu ultime lourd de ces opérations est stocké comme déchet dans un réservoir. La demande US 2,144,612 décrit un procédé de fabrication de dichloropropanol par réaction entre du glycérol et du chlorure d'hydrogène gazeux en présence d'un solvant non miscible à l'eau. La présence d'un tiers solvant complique les opérations de séparation des produits de la réaction. La demande WO 2005/054167 de SOLVAY SA décrit un procédé de fabrication de dichloropropanol par réaction entre du glycérol et du chlorure d'hydrogène en présence d'un acide organique de façon à obtenir des produits de réaction contenant du dichloropropanol. Dans ce procédé, on sépare souvent le dichloropropanol des autres produits de la réaction et on recycle ces derniers au réacteur de chloration du glycérol. Les autres produits de réaction peuvent contenir des composés à haut point d'ébullition qui ont tendance à s'accumuler dans le réacteur de chloration du glycérol. On peut soutirer une fraction de ces autres produits de réaction via une purge et soumettre cette fraction à différents traitements avant une éventuelle mise en décharge. La mise en décharge ne constitue pas une solution acceptable d'un point de vue environnemental. De plus, le surcoût lié au traitement préalable à la mise en décharge peut être prohibitif pour l'économie du procédé. Dans ce procédé, le glycérol n'ayant pas réagi est une cause de perte de sélectivité.

Le but de l'invention est de fournir un procédé de fabrication de dichloropropanol par chloration de glycérol qui ne présente pas ces inconvénients.

L'invention concerne donc un procédé de fabrication de dichloropropanol, dans lequel on soumet du glycérol, un ester de glycérol, ou un mélange d'entre eux, à une réaction avec un agent de chloration, en présence d'une phase liquide comprenant composés lourds autres que le glycérol et dont la température d'ébullition sous une pression de 1 bar absolu (100 kPa) est d'au moins 15 °C supérieure à la température d'ébullition du dichloropropanol sous une pression de 1 bar absolu (100 kPa), sélectionnés parmi les oligomères de glycérol partiellement chlorés et/ou estérifiés, et leurs mélanges, est dont la teneur est supérieure ou égale à 10 % en poids de la phase liquide et inférieure ou égale à 90 % en poids de la phase liquide.

On a trouvé de façon surprenante qu'en présence d'une quantité minimale de ces composés lourds dans l'étape de chloration, on pouvait améliorer le rendement du procédé. Sans vouloir être lié par une explication théorique, on pense que la formation principale de sous-produits non valorisables provient de l'oligomérisation glycérol et/ou de ses esters sur lui-même et que la présence et/ou le maintien de ces composés lourds dans l'étape de chloration permet de diluer le glycérol n'ayant pas réagi dans le milieu réactionnel sans affecter négativement le rendement de la réaction. On pense que la présence de ces composés permet concomitamment d'effectuer la réaction à une température plus élevée et d'ainsi compenser l'effet de la dilution du glycérol sur le rendement de la réaction et la productivité du procédé.

Les esters de glycérol peuvent être présents dans le glycérol et/ou être produits dans le procédé de fabrication du dichloropropanol et/ou être fabriqués préalablement au procédé de fabrication du dichloropropanol. Des exemples d'esters de glycérol comprennent les monoacétates de glycérol, les monostéarates de glycérol, les diacétates de glycérol et leurs mélanges.

Dans le procédé de fabrication selon l'invention, la réaction peut être menée en mode continu ou en mode discontinu. Le mode continu est préféré.

Le glycérol, l'ester de glycérol, ou le mélange d'entre eux, dans le procédé selon l'invention peuvent être obtenus au départ de matières premières fossiles ou au départ de matières premières renouvelables, de préférence au départ de matières premières renouvelables.

Par matières premières fossiles, on entend désigner des matières issues du traitement des ressources naturelles pétrochimiques, par exemple le pétrole, le gaz naturel, et le charbon. Parmi ces matières, les composés organiques comportant 3 atomes de carbone sont préférés. Le chlorure d'allyle, l'alcool allylique et le glycérol « synthétique » sont particulièrement préférés. Par glycérol « synthétique », on entend désigner un glycérol généralement obtenu à partir de ressources pétrochimiques.

Par matières premières renouvelables, on entend désigner des matières issues du traitement des ressources naturelles renouvelables. Le glycérol « naturel » est préférés. Du glycérol « naturel » est par exemple obtenus par conversion de sucres via des procédés thermochimiques, ces sucres pouvant être obtenus au départ de biomasse, comme décrit dans "Industrial Bioproducts : Today and Tomorrow, Energetics, Incorporated for the U.S. Department of Energy, Office of Energy Efficiency and Renewable Energy, Office of the Biomass Program, July 2003, pages 49, 52 to 56". Un de ces procédés est par exemple l'hydrogénolyse catalytique du sorbitol obtenu par conversion thermochimique du glucose. Un autre procédé est par exemple l'hydrogénolyse catalytique du xylitol obtenu par hydrogénation du xylose. Le xylose peut par exemple être obtenu par hydrolyse de l'hemicellulose contenue dans les fibres de maïs. Par « glycérol naturel » ou « glycérol obtenu à partir de matières premières renouvelables » on entend désigner en particulier du glycérol obtenu au cours de la fabrication de biodiesel ou encore du glycérol obtenu au cours de transformations de graisses ou huiles d'origine végétale ou animale en général telles que des réactions de saponification, de trans-estérification ou d'hydrolyse.

Parmi les huiles utilisables pour fabriquer le glycérol naturel, on peut citer toutes les huiles courantes, comme les huiles de palme, de palmiste, de coprah, de babassu, de colza ancien ou nouveau, de tournesol, de maïs, de ricin et de coton, les huiles d'arachide, de soja, de lin et de crambe et toutes les huiles issues par exemple des plantes de tournesol ou de colza obtenues par modification génétique ou hybridation.

On peut même utiliser des huiles de friture usagées, des huiles animales variées, comme les huiles de poisson, le suif, le saindoux et même des graisses d'équarrissage.

Parmi les huiles utilisées, on peut encore indiquer des huiles partiellement modifiées par exemple par polymérisation ou oligomérisation comme par exemple les "standolies" d'huiles de lin, de tournesol et les huiles végétales soufflées.

Un glycérol particulièrement adapté peut être obtenu lors de la transformation de graisses animales. Un autre glycérol particulièrement adapté peut être obtenu lors de la fabrication de biodiesel. Un troisième glycérol tout particulièrement bien adapté peut être obtenu lors de la transformation de graisses ou d'huiles, animales ou végétales, par trans-estérification en présence d'un catalyseur hétérogène, tel que décrit dans les documents FR 2752242,

FR 2869612 et FR 2869613. Plus spécifiquement, le catalyseur hétérogène est choisi parmi les oxydes mixtes d'aluminium et de zinc, les oxydes mixtes de zinc et de titane, les oxydes mixtes de zinc, de titane et d'aluminium, et les oxydes mixtes de bismuth et d'aluminium, et le catalyseur hétérogène est mis en oeuvre sous la forme d'un lit fixe. Ce dernier procédé peut être un procédé de fabrication de biodiesel.

Dans le procédé de fabrication de dichloropropanol selon l'invention, le glycérol peut être tel que décrit dans la demande de brevet intitulée « Procédé de préparation de chlorhydrine par conversion d'hydrocarbures aliphatiques poly hydroxylés » déposée au nom de SOLVAY SA le même jour que la présente demande.

Mention particulière est faite d'un procédé de fabrication de dichloropropanol dans lequel on fait réagir du glycérol, un ester de glycérol, ou un mélange d'entre eux, dont la teneur totale en métaux exprimés sous forme d'éléments est supérieure ou égale à 0,1 µg/kg et inférieure ou égale à 1 000 mg/kg, avec un agent de chloration.

Dans le procédé selon l'invention, on préfère utiliser du glycérol, un ester de glycérol, ou un mélange d'entre eux, obtenu au départ de matières premières renouvelables.

Dans le procédé de fabrication de dichloropropanol selon l'invention, le glycérol, l'ester de glycérol, ou le mélange d'entre eux, peut être un produit brut ou un produit épuré, tels que spécifiquement divulgués dans la demande WO 2005/054167 de SOLVAY SA, de la page 2, ligne 8, à la page 4, ligne 2.

Dans le procédé de fabrication de dichloropropanol selon l'invention, le glycérol peut être un glycérol dont la teneur en métaux alcalin et/ou alcalino-terreux peut être inférieure ou égale à 5 g/kg tel que décrit dans la demande intitulée « Procédé de fabrication d'une chlorhydrine par chloration d'un hydrocarbure aliphatique poly hydroxylé » déposée au nom de SOLVAY SA le même jour que la présente demande, dont le contenu est ici incorporé par référence. Les métaux alcalins peuvent être sélectionnés parmi le lithium, le sodium, le potassium, le rubidium et le césium et les métaux alcalino-terreux peuvent être sélectionnés parmi le magnésium, le calcium, le strontium et le barium.

Dans le procédé selon l'invention, la teneur en métaux alcalins et/ou alcalino-terreux du glycérol, de l'ester de glycérol, ou du mélange d'entre eux, est inférieure ou égale à 5 g/kg, souvent inférieure ou égale à 1 g/kg, plus particulièrement inférieure ou égale à 0,5 g/kg et dans certains cas inférieure ou égale à 0,01 g/kg. La teneur métaux alcalins et/ou alcalino-terreux du glycérol est généralement supérieure ou égale à 0,1 µg/kg.

Dans le procédé selon l'invention, les métaux alcalins sont généralement le lithium, le sodium, le potassium et le césium, souvent le sodium et le potassium, et fréquemment le sodium.

Dans le procédé de fabrication de dichloropropanol selon l'invention, la teneur en lithium du glycérol, de l'ester de glycérol, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en sodium glycérol de l'ester de glycérol, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en potassium du glycérol, de l'ester de glycérol, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en rubidium du glycérol, de l'ester de glycérol, ou du mélange d'entre eux, est généralement inférieure ou égale à 1 g/kg, souvent inférieure ou égale à 0,1 g/kg et plus particulièrement inférieure ou égale à 2mg/kg. Cette teneur est généralement supérieure ou égale à 0,1 µg/kg.

Dans le procédé selon l'invention, la teneur en césium du glycérol, de l'ester de glycérol, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, les éléments alcalino-terreux sont généralement le magnésium, le calcium, le strontium et le barium, souvent le magnésium et le calcium et fréquemment le calcium.

Dans le procédé selon l'invention, la teneur en magnésium du glycérol, de l'ester de glycérol, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en calcium du glycérol, de l'ester de glycérol, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en strontium du glycérol, de l'ester de glycérol, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, la teneur en barium du glycérol, de l'ester de glycérol, ou du mélange d'entre eux, est généralement inférieure ou égale à 1 g/kg, souvent inférieure ou égale à 0,1 g/kg et plus particulièrement inférieure ou égale à 2 mg/kg. Cette teneur est généralement supérieure ou égale à 0,1 µg/kg.

Dans le procédé selon l'invention, les métaux alcalins et/ou alcalino-terreux sont généralement présents sous la forme de sels, fréquemment sous la forme de chlorures, de sulfates et de leurs mélanges. Le chlorure de sodium est le plus souvent rencontré.

Dans le procédé de fabrication de dichloropropanol selon l'invention, l'agent de chloration peut être tel que décrit dans la demande WO 2005/054167 de SOLVAY SA, de la page 4, ligne 25, à la page 6, ligne 2.

Dans le procédé de fabrication de dichloropropanol selon l'invention, l'agent de chloration peut être du chlorure d'hydrogène peut être tel que décrit dans la demande WO 2005/054167 de SOLVAY SA, de la page 4, ligne 30, à la page 6, ligne 2.

Mention particulière est faite d'un agent de chloration qui peut être de l'acide chlorhydrique aqueux ou du chlorure d'hydrogène de préférence anhydre. Le chlorure d'hydrogène peut provenir d'un procédé de pyrolyse de composés organiques chlorés comme par exemple d'une fabrication de chlorure de vinyle, d'un procédé de fabrication de 4,4-méthylènediphenyl diisocyanate (MDI) ou dede toluène diisocyanate (TDI), de procédés de décapage des métaux ou d'une réaction entre un acide inorganique comme l'acide sulfurique ou phosphorique et un chlorure métallique tel que le chlorure de sodium, le chlorure de potassium ou le chlorure de calcium.

Dans un mode de réalisation avantageux du procédé de fabrication de dichloropropanol selon l'invention, l'agent de chloration est du chlorure d'hydrogène gazeux ou une solution aqueuse de chlorure d'hydrogène ou une combinaison des deux.

Dans le procédé de fabrication de dichloropropanol selon l'invention, le chlorure d'hydrogène peut être une solution aqueuse de chlorure d'hydrogène ou du chlorure d'hydrogène de préférence anhydre, issu d'une installation de fabrication de chlorure d'allyle et/ou de fabrication de chlorométhanes et/ou de chlorinolyse et/ou d'oxydation à haute température de composés chlorés tels que décrit dans la demande intitulée « Procédé de fabrication d'une chlorhydrine par réaction entre un hydrocarbure aliphatique poly hydroxylé et un agent de chloration » déposée au nom de SOLVAY SA le même jour que la présente demande, dont le contenu est ici incorporé par référence.

Mention particulière est faite d'un procédé de fabrication de dichloropropanol à partir de glycérol d'un ester de glycérol, ou d'un mélange d'entre eux, et d'un agent de chloration, ce dernier contenant au moins un des composés suivants : azote, oxygène, hydrogène, chlore, un composé organique hydrocarboné, un composé organique halogéné, un composé organique oxygéné et un métal.

Mention particulière est faite d'un composé organique hydrocarboné qui est choisi parmi les hydrocarbures aromatiques, aliphatiques saturés ou insaturés et leurs mélanges.

Mention particulière est faite d'un hydrocarbure aliphatique insaturé qui est choisi parmi l'acétylène, l'éthylène, le propylène, le butène, le propadiène, le méthylacétylène, et leurs mélanges, d'un hydrocarbure aliphatique saturé qui est choisi parmi le méthane, l'éthane, le propane, le butane, et leurs mélanges, et d'un hydrocarbure aromatique qui est le benzène.

Mention particulière est faite d'un composé organique halogéné qui est un composé organique chloré choisi parmi les chlorométhanes, les chloroéthanes, les chloropropanes, les chlorobutanes, le chlorure de vinyle, le chlorure de vinylidène, les monochloropropènes, le perchloroéthylène, le trichloréthylène, les chlorobutadiène, les chlorobenzènes et leurs mélanges.

Mention particulière est faite d'un composé organique halogéné qui est un composé organique fluoré choisi parmi les fluorométhanes, les fluoroéthanes, le fluorure de vinyle, le fluorure de vinylidène, et leurs mélanges.

Mention particulière est faite d'un composé organique oxygéné qui est choisi parmi les alcools, les chloroalcools, les chloroéthers et leurs mélanges

Mention particulière est faite d'un métal choisi parmi les métaux alcalins, les métaux alcalino-terreux, le fer, le nickel, le cuivre, le plomb, l'arsenic, le cobalt, le titane, le cadmium, l'antimoine, le mercure, le zinc, le sélénium, l'aluminium, le bismuth, et leurs mélanges.

Mention est plus particulièrement faite d'un procédé dans lequel l'agent de chloration est issu au moins partiellement d'un procédé de fabrication de chlorure d'allyle et/ou d'un procédé de fabrication de chlorométhanes et/ou d'un procédé de chlorinolyse et/ou d'un procédé d'oxydation de composés chlorés à une température supérieure ou égale à 800 °C.

Dans un mode de réalisation particulièrement avantageux du procédé de fabrication de dichloropropanol selon l'invention, le chlorure d'hydrogène est une solution aqueuse de chlorure d'hydrogène et ne comprend pas de chlorure d'hydrogène gazeux.

Dans le procédé de fabrication de dichloropropanol e selon l'invention, la réaction entre le glycérol, l'ester de glycérol, ou le mélange d'entre eux, et l'agent de chloration, peut être effectuée dans un réacteur tel que décrit dans la demande WO 2005/054167 de SOLVAY SA, à la page 6, lignes 3 à 23.

Mention est particulièrement faite d'une installation réalisée en, ou recouverte de, matériaux résistants dans les conditions de la réaction aux agents de chloration, en particulier au chlorure d'hydrogène. Mention est plus particulièrement faite d'une installation réalisée en acier émaillé ou en tantale.

Dans le procédé de fabrication de dichloropropanol selon l'invention, la réaction entre le glycérol, l'ester de glycérol, ou le mélange d'entre eux, et l'agent de chloration, peut être effectuée dans des équipements, réalisés en ou recouverts de, matériaux résistant aux agents de chloration, tels que décrit dans la demande intitulée « Procédé de fabrication d'une chlorhydrine dans des équipements résistant à la corrosion » déposée au nom de SOLVAY SA le même jour que la présente demande, dont le contenu est ici incorporé par référence.

Mention particulière est faite d'un procédé de fabrication de dichloropropanol comprenant une étape dans laquelle on soumet du glycérol, un ester de glycérol, ou un mélange d'entre eux, à une réaction avec un agent de chloration contenant du chlorure d'hydrogène et au moins une autre étape effectuée dans un équipement réalisé en ou recouvert de matériaux résistant à l'agent de chloration, dans les conditions de réalisation de cette étape. Mention est plus particulièrement faite de matériaux métalliques tels que l'acier émaillé, l'or et le tantale et de matériaux non-métalliques tels que le polyéthylène haute densité, le polypropylène, le poly(fluorure-de-vinylidène), le polytétrafluoroéthylène, les perfluoro alcoxyalcanes et le poly(perfluoropropylvinyléther), les polysulfones et les polysulfures, le graphite et le graphite imprégné.

Dans le procédé de fabrication de dichloropropanol selon l'invention, la réaction entre le glycérol et le chlorure d'hydrogène, peut être effectuée dans un milieu réactionnel, tel que décrit dans la demande intitulée « Procédé continu de fabrication de chlorhydrines » déposée au nom de SOLVAY SA le même jour que la présente demande.

Mention particulière est faite d'un procédé continu de production de dichloropropanol dans lequel on fait réagir du glycérol, un ester de glycérol ou un mélange d'entre eux, avec un agent de chloration et un acide organique dans un milieu réactionnel liquide dont la composition à l'état stationnaire comprend du glycérol et des esters de glycérol dont la somme des teneurs exprimée en mole de glycérol est supérieure à 1,1 mol % et inférieure ou égale à 30 mol %, le pourcentage étant rapporté à la partie organique du milieu réactionnel liquide.

La partie organique du milieu réactionnel liquide consiste en l'ensemble des composés organiques du milieu réactionnel liquide c'est-à-dire les composés dont la molécule contient au moins 1 atome de carbone.

Dans le procédé de fabrication de dichloropropanol selon l'invention, la réaction entre le glycérol, l'ester de glycérol, ou le mélange d'entre eux, et agent de chloration, peut être effectuée en présence d'un catalyseur tel que décrit dans la demande WO 2005/054167 de SOLVAY SA, de la page 6, ligne 28, à la page 8, ligne 5.

Mention est particulièrement faite d'un catalyseur basé sur un acide carboxylique ou sur un dérivé d'acide carboxylique ayant un point d'ébullition atmosphérique supérieur ou égal à 200 °C, en particulier l'acide adipique et les dérivés de l'acide adipique.

Le procédé de fabrication d'une chlorhydrine selon l'invention, peut être réalisé en présence d'un acide organique.

L'acide organique peut être un produit provenant du procédé de fabrication du glycérol ou un produit ne provenant pas de ce procédé. Dans ce dernier cas, il peut s'agir d'un acide organique utilisé pour catalyser la réaction entre le glycérol et l'agent de chloration et/ou d'un acide généré dans le procédé de fabrication du dichloropropanol. On pense par exemple à des acides générés au départ d'aldéhydes présents dans le glycérol ou formés lors de la fabrication du dichloropropanol. L'acide organique peut aussi être un mélange d'acide organique provenant du procédé de fabrication du glycérol et d'un acide organique ne provenant pas du procédé de fabrication du glycérol.

Dans le procédé selon l'invention, les esters de de glycérol peuvent provenir de la réaction entre le glycérol et l'acide organique, avant, pendant ou dans les étapes qui suivent la réaction avec l'agent de chloration.

Dans le procédé de fabrication de dichloropropanol selon l'invention, la réaction entre le glycérol, l'ester de glycérol ou le mélange d'entre eux, et l'agent de chloration peut être effectuée à une concentration en catalyseur, une température, à une pression et pour des temps de séjour tels que décrits dans la demande WO 2005/054167 de SOLVAY SA, de la page 8, ligne 6 à la page 10, ligne 10.

Mention est particulièrement faite d'une température d'au moins 20 °C et d'au plus 160 °C, d'une pression d'au moins 0,3 bar (30 kPa) et d'au plus, 100 bar (10 MPa), et d'un temps de séjour d'au moins 1 h et d'au plus 50 h.

Dans le procédé de fabrication du dichloropropanol selon l'invention, la réaction entre le glycérol, l'ester de glycérol ou le mélange d'entre eux, et l'agent de chloration peut être effectuée en présence d'un solvant tel que décrit dans la demande WO 2005/054167 de SOLVAY SA, à la page 11, lignes 12 à 36.

Mention est particulièrement faite d'un solvant organique tel qu'un solvant organique chloré, un alcool, une cétone, un ester ou un éther, un solvant non aqueux miscible avec le glycérol tel que le chlroéthanol, le chloropropanol, le chloropropanediol, le dichloropropanol, le dioxanne, le phénol, le crésol, et les mélanges de chloropropanediol et de dichloropropanol, ou des produits lourds, de la réaction tels que les oligomères de glycérol partiellement chlorés et ou estérifiés.

Dans le procédé de fabrication du dichloropropanol selon l'invention, la réaction entre le glycérol, l'ester de glycérol, ou le mélange d'entre eux, et l'agent de chloration est préférentiellement effectuée dans un milieu réactionnel liquide. Le milieu réactionnel liquide peut être mono-ou multiphasique.

Le milieu réactionnel liquide est constitué par l'ensemble des composés solides dissous ou dispersés, liquides dissous ou dispersés et gazeux dissous ou dispersés, à la température de la réaction.

Le milieu réactionnel comprend les réactifs, le catalyseur, le solvant, les impuretés présentes dans les réactifs, dans le solvant et dans le catalyseur, les intermédiaires de réaction, les produits et les sous-produits de la réaction.

Par réactifs, on entend désigner le glycérol, l'ester de glycérol et l'agent de chloration.

Parmi les impuretés présentes dans le glycérol, on peut citer les acides carboxyliques, les sels d'acides carboxyliques, les esters d'acide gras avec le glycérol, les esters d'acides gras avec les alcools utilisés lors de la trans-estérification, les sels inorganiques tels que les chlorures et les sulfates alcalins ou alcalino-terreux.

On peut citer parmi les impuretés du glycérol les acides carboxyliques, les sels d'acides carboxyliques, les esters d'acide gras tels que les mono-, les di- et les triglycérides, les esters d'acides gras avec les alcools utilisés lors de la trans-estérification, les sels inorganiques tels que les chlorures et les sulfates alcalins ou alcalino-terreux.

Parmi les intermédiaires réactionnels on peut citer les monochlorhydrines de glycérol et leurs esters et/ou polyesters, les esters et/ou polyesters de glycérol et les esters des polychlorhydrines.

On peut citer parmi les intermédiaires réactionnels, la monochlorhydrine de glycérol et ses esters et/ou polyesters, les esters et/ou polyesters de glycérol et les esters de dichloropropanol.

L'ester de glycérol peut donc être selon le cas, un réactif, une impureté du glycérol ou un intermédiaire réactionnel.

Par produits de la réaction, on entend désigner le dichloropropanol et l'eau. L'eau peut être l'eau formée dans la réaction de chloration et/ou de l'eau introduite dans le procédé, par exemple via le glycérol et/ou l'agent de chloration, tel que décrit dans la demande WO 2005/054167 de SOLVAY SA, à la page 2, lignes 22 à 28, à la page 3, lignes 20 à 25, à la page 5, lignes 7 à 31 et à la page 12, lignes 14 à 19.

Parmi les sous-produits, on peut citer par exemple, les oligomères de glycérol partiellement chlorés et/ou estérifiés.

Par sous-produits non-valorisables, on entend désigner des produits issus du glycérol ou de ses dérivés qui dans les conditions de réaction ne permettent pas de générer de dichloropropanol. Les oligomères de glycérol partiellement chlorés et/ou estérifiés sont des exemples de sous-produits non-valorisables.

Parmi les sous-produits non-valorisables, on peut citer par exemple, les oligomères du glycérol partiellement chlorés et/ou estérifiés.

Les intermédiaires réactionnels et les sous-produits peuvent être formés dans les différentes étapes du procédé comme par exemple, au cours de l'étape de fabrication du dichloropropanol et au cours des étapes de séparation du dichloropropanol.

Le milieu réactionnel liquide peut ainsi contenir le glycérol, l'agent de chloration dissous ou dispersé sous forme de bulles, le catalyseur, le solvant, les impuretés présentes dans les réactifs, le solvant et le catalyseur, comme des sels dissous ou solides par exemple, le solvant, le catalyseur, les intermédiaires réactionnels, les produits et les sous-produits de la réaction.

Le procédé selon l'invention peut être effectué en mode batch ou en mode continu. Le mode continu est préféré.

Les composés lourds ont une température d'ébullition sous une pression de 1 bar absolu (100 kPa) supérieure ou égale à 200 °C, de préférence supérieure ou égale à 220 °C, de manière plus préférée supérieure ou égale à 240 °C et de manière tout particulièrement préférée supérieure ou égale à 250 °C.

Dans une opération de séparation par distillation en présence d'eau et de dichloropropanol par exemple, ces composés lourds sortent en pied de la colonne de distillation tandis que l'eau et le dichloropropanol sortent en tête de la colonne de distillation.

La teneur de la phase liquide en composés lourds, est supérieure ou égale à 10 % en poids de la phase liquide, de préférence supérieure ou égale à 15 % en poids et de manière particulièrement préférée supérieure ou égale à 20 % en poids. Cette teneur est inférieure ou égale à 90 % en poids, de préférence inférieure ou égale à 80 % en poids et de manière particulièrement préférée inférieure ou égale à 75 % en poids de la phase liquide.

Les composés lourds peuvent être « externes » ou « internes » au procédé selon l'invention. Par l'expression « externe », on entend désigner des composés lourds qui n'ont pas été produits dans le procédé selon l'invention, . Par l'expression « interne », on entend désigner des composés lourds qui sont des produits formés dans le procédé selon l'invention. Ces produits peuvent résulter de réactions secondaires entre le glycérol, l'agent de chloration, les produits de la réaction de chloration et les acides présents dans glycérol et/ou utilisés comme catalyseurs de la réaction. Les composés lourds peuvent être considérés comme un constituant du solvant. L'utilisation de ces composés lourds « internes » au procédé offre l'avantage supplémentaire de limiter la présence d'un tiers solvant dans le procédé, ce qui simplifie les étapes de séparation.

Les composés lourds sont sélectionnés parmi les les oligomères de glycérol partiellement chlorés et/ou estérifiés et leurs mélanges . Ces oligomères de GLC peuvent être linéaires ou cycliques.

Dans le procédé selon l'invention, au moins une fraction des composés lourds a été séparée des autres composés du milieu réactionnel, notamment du dichloropropanol et a ensuite été recyclée dans la réaction entre le glycérol et l'agent de chloration.

Dans un premier mode de réalisation du procédé selon l'invention, on utilise des composés lourds qui sont externes au procédé selon l'invention.

Dans un deuxième mode de réalisation du procédé selon l'invention qui est préféré, on utilise des composés lourds qui sont internes au procédé selon l'invention.

Dans un troisième mode de réalisation du procédé selon l'invention, on utilise un mélange de composés lourds internes et externes au procédé selon l'invention.

Dans le procédé selon l'invention, la séparation du dichloropropanol et des autres composés du milieu réactionnel peut être effectuée selon les modes tels que décrits dans la demande WO 2005/054167 de SOLVAY SA, de la page 12, ligne 1, à la page 16, ligne 35 et à la page 18, lignes 6 à 13. Ces autres composés sont ceux mentionnés ci-dessus et comprennent les réactifs non consommés, les impuretés présentes dans les réactifs, le catalyseur, le solvant, les intermédiaires réactionnels, l'eau et les sous produits de la réaction.

Mention particulière est faite d'une séparation par distillation azéotropique d'un mélange eau dichloropropanol /agent de chloration dans des conditions minimisant les pertes en agent de chloration suivie d'une séparation de la chlorhydrine par décantation.

Dans le procédé de fabrication de dichloropropanol selon l'invention, la séparation du dichloropropanol et des autres composés du milieu réactionnel, peut être effectuée selon des modes tels que décrits dans la demande de brevet EP 05104321.4 déposée au nom de SOLVAY SA le 20/05/2005. Mention particulière est faite d'un mode de séparation comprenant au moins une opération de séparation destinée à enlever le sel de la phase liquide.

Mention particulière est faite d'un procédé de fabrication de dichloropropanol par réaction entre glycérol, un ester de glycérol ou un mélange d'entre eux, et un agent de chloration dans lequel le glycérol, un ester de glycérol ou un mélange d'entre eux, utilisé contient au moins un sel métallique solide ou dissous, le procédé comprenant une opération de séparation destinée à enlever une partie du sel métallique. Mention est plus particulièrement est faite d'un procédé de fabrication de dichloropropanol par réaction entre un du glycérol, un ester de glycérol ou un mélange d'entre eux, et un agent de chloration dans lequel le glycérol, on ester de glycérol ou un mélange d'entre eux, utilisé contient au moins un chlorure et/ou un sulfate de sodium et/ou potassium et dans lequel l'opération de séparation destinée à enlever une partie du sel métallique est un opération de filtration. Mention est aussi particulièrement faite d'un procédé de fabrication de dichloropropanol dans lequel (a) on soumet du glycérol, un ester de glycérol ou un mélange d'entre eux, à une réaction avec un agent de chloration dans un milieu réactionnel, (b) on prélève en continu ou périodiquement une fraction du milieu réactionnel contenant au moins de l'eau et du dichloropropanol, (c) au moins une partie de la fraction obtenue à l'étape (b) est introduite dans une étape de distillation et (d) le taux de reflux de l'étape de distillation est contrôlé en fournissant de l'eau à ladite étape de distillation. Mention est tout particulièrement faite d'un procédé de fabrication de dichloropropanol dans lequel (a) on soumet du glycérol, un ester de glycérol ou un mélange d'entre eux, à une réaction avec du chlorure d'hydrogène dans un milieu réactionnel, (b) on prélève en continu ou périodiquement une fraction du milieu réactionnel contenant au moins de l'eau et du dichloropropanole, (c) au moins une partie de la fraction obtenue à l'étape (b) est introduite dans une étape de distillation, dans lequel le rapport entre la concentration en chlorure d'hydrogène et la concentration en eau dans la fraction introduite dans l'étape de distillation est plus petit que le rapport de concentrations chlorure d'hydrogène/eau dans la composition binaire azéotropique chlorure d'hydrogène/eau à la température et à la pression de distillation.

Dans le procédé de fabrication du dichloropropanol selon l'invention, la séparation du dichloropropanol et des autres composés du milieu réactionnel de chloration du glycérol peut être effectuée selon les modes tels que décrits dans la demande intitulée « Procédé de fabrication d'une chlorhydrine » déposée au nom de SOLVAY SA, le même jour que la présente demande.

Mention particulière est faite d'un procédé de fabrication de dichloropropanol comprenant les étapes suivantes (a) on fait réagir du glycérol, un ester de glycérol, ou un mélange d'entre eux, avec un agent de chloration et un acide organique de façon à obtenir un mélange contenant du dichloropropanol et des esters de dichloropropanol, (b) on soumet au moins une partie du mélange obtenu à l'étape (a) à un ou plusieurs traitements dans des étapes ultérieures à l'étape (a)et (c) on ajoute du glycérol à au moins une des étapes ultérieures à l'étape (a), pour qu'il réagisse à une température supérieure ou égale à 20 °C, avec les esters de dichloropropanol de façon à former au moins partiellement des esters de glycérol.

Dans le procédé de fabrication de dichloropropanol selon l'invention, la séparation du dichloropropanol et des autres composés du milieu réactionnel de chloration du glycérol peut être effectuée selon les modes tels que décrits dans la demande intitulée « Procédé de fabrication d'une chlorhydrine au départ d'un hydrocarbure aliphatique poly hydroxylé » déposée au nom de SOLVAY SA le même jour que la présente demande.

Mention particulière est faite d'un procédé de fabrication de dichloropropanol par réaction entre du glycérol, un ester de glycérol, ou un mélange d'entre eux, et un agent de chloration dans un réacteur qui est alimenté en un ou plusieurs flux liquides contenant moins de 50 % en poids de glycérol, de l'ester de glycérol ou du mélange d'entre eux, par rapport au poids de la totalité des flux liquides introduits dans le réacteur. Mention plus particulière est faite d'un procédé comprenant les étapes suivantes : (a) on fait réagir du glycérol, un ester de glycérol, ou un mélange d'entre eux, avec un agent de chloration de façon à obtenir au moins un milieu contenant du dichloropropanol, de l'eau et de l'agent de chloration, (b) on prélève au moins une fraction du milieu formé à l'étape (a) et (c) on soumet la fraction prélevée à l'étape (b) à une opération de distillation et/ou de stripping dans laquelle on ajoute du glycérol de façon à séparer de la fraction prélevée à l'étape (b) un mélange contenant de l'eau et du dichloropropanol présentant une teneur réduite en agent de chloration comparée à celle de la fraction prélevée à l'étape (b).

Dans le procédé de fabrication du dichloropropanol selon l'invention, la séparation du dichloropropanol et des autres composés du milieu réactionnel de chloration du glycérol peut être effectuée selon les modes tels que décrits dans la demande intitulée « Procédé de conversion d'hydrocarbures aliphatiques poly hydroxylés en chlorhydrines » déposée au nom de SOLVAY SA le même jour que la présente demande. Mention particulière est faite d'un procédé de préparation de dichloropropanol comprenant les étapes suivantes : (a) on fait réagir du glycérol, un ester de glycérol, ou un mélange d'entre eux, avec un agent de chloration de façon à obtenir un mélange contenant duy dichloropropanol, des esters de dichloropropanol et de l'eau, (b) on soumet au moins une fraction du mélange obtenu à l'étape (a) à un traitement de distillation et/ou de stripping de façon à obtenir une partie concentrée en eau, en dichloropropanol et en esters de dichloropropanol et (c) on soumet au moins une fraction de la partie obtenue à l'étape (b) à une opération de séparation en présence d'au moins un additif de façon à obtenir une portion concentrée en dichloropropanol et en esters de dichloropropanol et qui contient moins de 40 % en poids d'eau.

L'opération de séparation est plus particulièrement une décantation. Dans le procédé de fabrication de dichloropropanol selon l'invention, la séparation et le traitement des autres composés du milieu réactionnel peuvent être effectués selon des modes tels que décrits dans la demande intitulée « Procédé de fabrication d'une chlorhydrine par chloration d'un hydrocarbure aliphatique poly hydroxylé » déposée au nom de SOLVAY SA le même jour que la présente demande. Un traitement préféré consiste à soumettre une fraction des sous-produits de la réaction à une oxydation à haute température.

Mention particulière est faite d'un procédé de fabrication de dichloropropanol comprenant les étapes suivantes (a) on fait réagir du glycérol, un ester de glycérol, ou un mélange d'entre eux, dont la teneur en métaux alcalins et/ou alcalino-terreux est inférieure ou égale à 5 g/kg, un agent oxydant et un acide organique de façon à obtenir un mélange contenant au moins du dichloropropanol et des sous-produits, (b) on soumet au moins une partie du mélange obtenu à l'étape (a) à un ou plusieurs traitements dans des étapes ultérieures à l'étape (a) et (c) au moins une des étapes ultérieures à l'étape (a), consiste en une oxydation à une température supérieure ou égale à 800 °C. Mention plus particulière est faite d'un procédé dans lequel dans l'étape ultérieure, on prélève une partie du mélange obtenu à l'étape (a) et on soumet cette partie à une oxydation à une température supérieure ou égale à 800 °C, pendant le prélèvement. Mention particulière est aussi faite d'un procédé dans lequel le traitement de l'étape (b) est une opération de séparation choisie parmi les opérations de décantation, de filtration, de centrifugation, d'extraction, de lavage, d'évaporation, de stripping, de distillation, d'adsorption ou les combinaisons d'au moins deux d'entre-elles.

Dans le procédé selon l'invention, le dichloropropanol, est généralement obtenu sous la forme d'un mélange de composés comprenant les isomères de 1,3-dichloropropane-2-ol et de 2,3-dichloropropane-1-ol. Ce mélange contient généralement plus de 1 % en poids des deux isomères, de préférence plus de 5 % en poids et de manière particulière plus de 50 %. Le mélange contient usuellement moins de 99,9 % en poids des deux isomères, de préférence moins de 95 % en poids et tout particulièrement moins de 90 % en poids. Les autres constituants du mélange peuvent être des composés provenant des procédés de fabrication du dichloropropanol, tels que des réactifs résiduels, des sous-produits de réaction, des solvants et notamment de l'eau.

Le rapport massique entre les isomères 1,3-dichloropropane-2-ol et 2,3-dichloropropane-1-ol est usuellement supérieur ou égal à 0,01, souvent, supérieur ou égal 0,4, fréquemment supérieur ou égal à 1,5, de préférence supérieur à ou égal à 3,0, de manière plus préférée supérieur ou égal à 7,0 et de manière tout particulièrement préférée supérieur ou égal à 20,0. Ce rapport est usuellement inférieur ou égal à 99 et de préférence inférieur ou égal à 25.

Le dichloropropanol obtenue dans le procédé selon l'invention peut contenir une teneur élevée en cétones halogénées, en particulier en chloroacétone, comme décrit dans la demande de brevet FR 05.05120 du 20/05/2005 déposée au nom de la demanderesse. La teneur en cétone halogénée peut être réduite en soumettant le dichloropropanol obtenue dans le procédé selon l'invention à une distillation azéotropique en présence d'eau ou en soumettant le dichloropropanol à un traitement de déshydrochloration comme décrit dans cette demande, de la page 4, ligne 1, à la page 6, ligne 35.

Mention particulière est faite d'un procédé de fabrication d'un époxyde dans lequel des cétones halogénées sont formées comme sous-produits et qui comprend au moins un traitement d'élimination d'au moins une partie des cétones halogénées formées. Mention est plus particulièrement faite d'un procédé de fabrication d'épichlorhydrine par déshydrochloration de dichloropropanol dont au moins une fraction est fabriquée par chloration de glycérol, d'un ester de glycérol, ou d'un mélange d'entre eux, d'un traitement de déshydrochloration et d'un traitement par distillation azéotropique d'un mélange eau-cétone halogénée destinés à éliminer au moins une partie des cétones halogénées formées et d'un procédé de fabrication d'épichlorhydrine dans lequel la cétone halogénée formée est la chloroacétone.

Le dichloropropanol obtenue dans le procédé selon l'invention peut être soumise à une réaction de déshydrochloration pour produire de l'épichlorhydrine comme décrit dans les demandes de brevet WO 2005/054167 et FR 05.05120 déposées au nom de SOLVAY SA

La déshydrochloration du dichloropropanol peut être effectuée comme décrit dans la demande intitulée « Procédé de fabrication d'un époxyde au départ d'un hydrocarbure aliphatique poly hydroxylé et d'un agent de chloration » déposée au nom de SOLVAY SA le même jour que la présente demande.

Mention particulière est faite d'un procédé de fabrication d'épichlorhydrine dans lequel on soumet un milieu réactionnel résultant de la réaction entre du glycérol, un ester de glycérol, ou un mélange d'entre eux, et un agent de chloration, le milieu réactionnel contenant au moins 10 g de dichloropropanol par kg de milieu réactionnel, à une réaction chimique ultérieure sans traitement intermédiaire.

Mention est également faite de fabrication d'épichlorhydrine comprenant les étapes suivantes : (a) on fait réagir du glycérol, un ester de glycérol, ou un mélange d'entre eux, avec un agent de chloration et un acide organique de façon à former du dichloropropanol et des esters de dichloropropanol dans un milieu réactionnel contenant du glycérol, de l'ester de glycérol, de l'eau, l'agent de chloration et l'acide organique, le milieu réactionnel contenant au moins 10 g de dichloropropanol par kg de milieu réactionnel, (b) on soumet au moins une fraction du milieu réactionnel obtenu à l'étape (a), fraction qui a la même composition que le milieu réactionnel obtenu à l'étape (a), à un ou plusieurs traitements dans des étapes ultérieures à l'étape (a) et (c) on ajoute un composé basique à au moins une des étapes ultérieures à l'étape (a) pour qu'il réagisse au moins partiellement avec le dichloropropanol, les esters de dichloropropanol, l'agent de chloration et l'acide organique de façon à former de l'épichlorhydrine et des sels.

Le procédé de fabrication du dichloropropanol selon l'invention peut être intégré dans un schéma global de fabrication d'épichlorhydrine tel que décrit dans la demande intitulée « Procédé de fabrication d'un époxyde au départ d'une chlorhydrine » déposée au nom de SOLVAY SA le même jour que la présente demande.

Mention particulière est faite d'un procédé de fabrication d'épichlorhydrine comprenant au moins une étape de purification de l'épichlorhydrine formée, l'épichlorhydrine étant au moins en partie fabriqué par un procédé de déshydrochloration de dichloropropanol, celui-ci étant au moins en partie fabriquée par un procédé de chloration de glycérol. d'un ester de glycérol, ou d'un mélange d'entre eux.

Le procédé selon l'invention peut être suivi d'une fabrication d'épichlorhydrine par déshydrochloration de dichloropropanol et l'épichlorhdyrine peut entrer dans la fabrication de résines époxy.

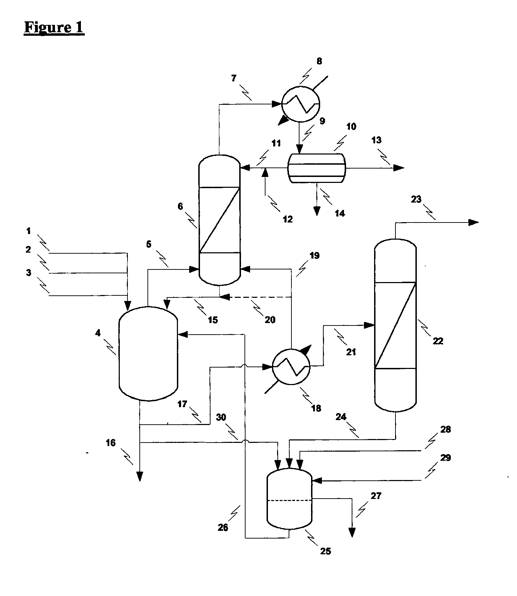

La Figure 1 montre un schéma particulier d'installation utilisable pour mettre en oeuvre le procédé selon l'invention.

Un réacteur (4) est alimenté en mode continu ou en mode batch avec du glycérol, un ester de glycérol, ou un mélange d'entre eux, via la ligne (1) et en catalyseur via la ligne (2), l'alimentation en agent de chloration est réalisée en mode continu ou en mode batch via la ligne (3), une colonne de distillation (6) est alimentée via la ligne (5) avec des vapeurs produites dans le réacteur (4), un flux est soutiré de la colonne (6) via la ligne (7) et est introduit dans un condenseur (8), le flux issu du condenseur est introduit via la ligne (9) dans un décanteur (10) dans lequel des phases aqueuses et organiques sont séparées. Une fraction de la phase aqueuse séparée est optionnellement recyclée via la ligne (11) au sommet de la colonne pour maintenir le reflux. De l'eau fraîche peut être introduite dans la ligne (11) via la ligne (12). La production de dichloropropanol est distribuée entre la phase organique soutirée via la ligne (14) et la phase aqueuse soutirée via la ligne (13). Le résidu de la colonne (6) peut être recyclé au réacteur (4) via la ligne (15). Une fraction des produits lourds est soutirée du réacteur (4) via la purge (16) et est introduite via la ligne (17) dans un évaporateur (18) dans lequel une opération partielle d'évaporation est menée par exemple par chauffage ou par balayage gazeux avec de l'azote ou de la vapeur d'eau, la phase gazeuse contenant la plus majeure partie de l'agent de chloration du flux (17) est recyclée via la ligne (19) à la colonne (6) ou via la ligne (20) au réacteur (4), une colonne de distillation ou de stripping (22) est alimentée avec la phase liquide en provenance de l'appareil de stripping (18) via la ligne (21), la majeure partie du dichloropropanol est recueillie au sommet de la colonne (22) via la ligne (23) et le résidu est introduit via la ligne (24) dans la colonne de filtration (25) dans laquelle des phases liquides et solides sont séparées, la phase liquide est recyclée via la ligne (26) au réacteur (4). Le solide peut être soutiré de l'unité de filtration (25) via la ligne (27) sous la forme d'un solide ou d'une solution. Des solvants peuvent être ajoutés à l'unité de filtration (25) via les lignes (28) et (29) pour le lavage et/ou la dissolution du solide et soutirés via la ligne (29). Optionnellement, un flux est soutiré de la purge (16) et introduit via la ligne (30) dans la colonne de filtration (25). L'évaporateur (18) et la colonne de distillation (22) sont alors court-circuités.

Les exemples ci-après entendent illustrer l'invention sans toutefois la limiter.

### Exemple 1 (conforme à l'invention)

Dans un réacteur contenant 800 g de glycérol, 63 g d'acide adipique, et 500 g d'un mélange constitué de 350 g de diglycérol dichloré et de 150 g de monochlorhydrine de glycérine maintenu à une température de 120 °C à pression atmosphérique, on fait barboter du chlorure d'hydrogène gazeux à un débit de 1,26 g/min pendant 10 h. L'eau de réaction est éliminée par distillation continue de l'azéotrope formé avec le 1,3-DCPol. On obtient au total 1207 g de dichloropropanol, 327 g d'eau et 69 g de chlorure d'hydrogène. La perte mesurée en composés oligomères (essentiellement diglycérine et diglycérine cyclique chlorées) s'élève à 0,7 % de la glycérine mise en oeuvre.

### Exemple 2 (non-conforme à l'invention)

Dans un réacteur contenant 800 g de glycérol et 63 g d'acide adipique, et maintenu à une température de 120 °C et à une pression de 1 bar (100 kPa); on fait barboter du chlorure d'hydrogène gazeux à un débit de 1,26 g/min pendant 10 h. L'eau de réaction est éliminée par distillation continue de l'azéotrope formé avec le 1,3-DCPol. On obtient au total 998 g de dichloropropanol, 296 g d'eau et 171 g de chlorure d'hydrogène. La perte mesurée en composés oligomères s'élève à 3 % de la glycérine mise en oeuvre.

## Revendications

1. Procédé de fabrication de dichloropropanol, dans lequel on soumet du glycérol, un ester de glycérol, ou un mélange d'entre eux, à une réaction avec un agent de chloration, en présence d'une phase liquide comprenant des composés lourds autres que le glycérol, dont la température d'ébullition sous une pression de 1 bar absolu est d'au moins 15 °C supérieure à la température d'ébullition du dichloropropanol sous une pression de 1 bar absolu (100 kPa), sélectionnés parmi les oligomères de glycérol partiellement chlorés et/ou estérifiés, et leurs mélanges, et dont la teneur est supérieure ou égale à 10 % en poids de la phase liquide et inférieure ou égale à 90 % en poids de la phase liquide.

2. Procédé selon la revendication 1 dans lequel la teneur en composés lourds est supérieure ou égale à 15 % en poids de la phase liquide.

3. Procédé selon la revendication 2 dans lequel la teneur en composés lourds est supérieure ou égale à 20 % en poids de la phase liquide.

4. Procédé selon l'une quelconque des revendications 1 à 3, dans lequel les composés lourds autres que le glycérol ont une température d'ébullition sous une pression de 1 bar absolu (100 kPa) supérieure ou égale à 220 °C.

5. Procédé selon la revendication 4, dans lequel les composés lourds autres que le glycérol ont une température d'ébullition sous une pression de 1 bar absolu (100 kPa) supérieure ou égale à 240 °C.

6. Procédé selon la revendication 5, dans lequel les composés lourds autres que le glycérol ont une température d'ébullition sous une pression de 1 bar absolu (100 kPa) supérieure ou égale à 250 °C.

7. Procédé selon l'une quelconque des revendications 1 à 6 dans lequel la réaction entre le glycérol, l'ester de glycérol, ou le mélange d'entre eux, et l'agent de chloration se produit dans la phase liquide.

8. Procédé selon l'une quelconque des revendications 1 à 7 dans lequel la réaction est menée en continu.

9. Procédé selon l'une quelconque des revendications 1 à 8 dans lequel une fraction des composés lourds est séparée du dichloropropanol et recyclée dans la réaction du glycérol, de l'ester de glycérol, ou du mélange d'entre eux, avec l'agent de chloration.

10. Procédé selon l'une quelconque des revendications 1 à 9 dans lequel une fraction des composés lourds est formée au cours de la réaction de chloration du glycérol, de l'ester de glycérol, ou du mélange d'entre eux.

11. Procédé selon l'une quelconque des revendications 1 à 10, dans lequel un acide carboxylique et/ou un dérivé d'acide carboxylique est utilisé comme catalyseur.

12. Procédé selon l'une quelconque des revendications 1 à 11 dans lequel le glycérol, l'ester de glycérol, ou le mélange d'entre eux, est obtenu au départ de matières premières renouvelables.

13. Procédé selon l'une quelconque des revendications 1 à 12 suivi d'une fabrication d'épichlorhydrine par déshydrochloration du dichloropropanol

14. Procédé selon la revendication 13 dans lequel l'épichlorhydrine entre dans la fabrication de résines époxy.

15. Procédé selon l'une quelconque des revendications 1 à 18 dans lequel l'agent de chloration est une combinaison de chlorure d'hydrogène gazeux et d'une solution aqueuse de chlorure d'hydrogène ou une solution aqueuse de chlorure d'hydrogène.

## Patentansprüche

1. Verfahren zur Herstellung von Dichlorpropanol, bei dem man Glycerin, einen Glycerinester oder eine Mischung davon einer Reaktion mit einem Chlorierungsmittel in Gegenwart einer flüssigen Phase, die von Glycerin verschiedene schwere Verbindungen, deren Siedetemperatur unter einem Druck von 1 bar absolut mindestens 15°C über der Siedetemperatur von Dichlorpropanol unter einem Druck von 1 bar absolut (100 kPa) liegt, die aus teilweise chlorierten und/oder veresterten Glycerinoligomeren und Mischungen davon ausgewählt sind und deren Gehalt größer gleich 10 Gew.-%, bezogen auf die flüssige Phase, und kleiner gleich 90 Gew.-%, bezogen auf die flüssige Phase, ist, umfasst, unterwirft.

2. Verfahren nach Anspruch 1, bei dem der Gehalt an schweren Verbindungen größer gleich 15 Gew.-%, bezogen auf die flüssige Phase, ist.

3. Verfahren nach Anspruch 2, bei dem der Gehalt an schweren Verbindungen größer gleich 20 Gew.-%, bezogen auf die flüssige Phase, ist.

4. Verfahren nach einem der Ansprüche 1 bis 3, bei dem die von Glycerin verschiedenen schweren Verbindungen eine Siedetemperatur unter einem Druck von 1 bar absolut (100 kPa) größer gleich 220°C aufweisen.

5. Verfahren nach Anspruch 4, bei dem die von Glycerin verschiedenen schweren Verbindungen eine Siedetemperatur unter einem Druck von 1 bar absolut (100 kPa) größer gleich 240°C aufweisen.

6. Verfahren nach Anspruch 5, bei dem die von Glycerin verschiedenen schweren Verbindungen eine Siedetemperatur unter einem Druck von 1 bar absolut (100 kPa) größer gleich 250°C aufweisen.

7. Verfahren nach einem der Ansprüche 1 bis 6, bei dem die Reaktion zwischen dem Glycerin, dem Glycerinester oder der Mischung davon und dem Chlorierungsmittel in der flüssigen Phase stattfindet.

8. Verfahren nach einem der Ansprüche 1 bis 7, bei dem die Reaktion kontinuierlich geführt wird.

9. Verfahren nach einem der Ansprüche 1 bis 8, bei dem eine Fraktion von schweren Verbindungen von dem Dichlorpropanol abgetrennt und in die Reaktion des Glycerins, des Glycerinesters oder der Mischung davon mit dem Chlorierungsmittel zurückgeführt wird.

10. Verfahren nach einem der Ansprüche 1 bis 9, bei dem im Lauf der Chlorierungsreaktion des Glycerins, des Glycerinesters oder der Mischung davon eine Fraktion von schweren Verbindungen gebildet wird.

11. Verfahren nach einem der Ansprüche 1 bis 10, bei dem eine Carbonsäure und/oder ein Carbonsäure derivat als Katalysator verwendet wird.

12. Verfahren nach einem der Ansprüche 1 bis 11, bei dem das Glycerin, der Glycerinester oder die Mischung davon aus erneuerbaren Ausgangsstoffen erhalten werden.

13. Verfahren nach einem der Ansprüche 1 bis 12, gefolgt von einer Herstellung von Epichlorhydrin durch Dehydrochlorierung des Dichlorpropanols.

14. Verfahren nach Anspruch 13, bei dem das Epichlorhydrin bei der Herstellung von Epoxidharzen verwendet wird.

15. Verfahren nach einem der Ansprüche 1 bis 18, bei dem es sich bei dem Chlorierungsmittel um eine Kombination von gasförmigem Chlorwasserstoff und einer wässrigen Lösung von Chlorwasserstoff oder eine wässrige Lösung von Chlorwasserstoff handelt.

## Claims

1. Process for the manufacture of dichloropropanol, in which glycerol, a glycerol ester or a mixture of them is subjected to a reaction with a chlorinating agent in the presence of a liquid phase comprising heavy compounds other than glycerol, the boiling point of which under a pressure of 1 bar absolute is at least 15°C greater than the boiling point of dichloropropanol under a pressure of 1 bar absolute (100 kPa), which compounds are selected from partially chlorinated and/or esterified glycerol oligomers, and their mixtures, and the content of which is greater than or equal to 10% by weight of the liquid phase and less than or equal to 90% by weight of the liquid phase.

2. Process according to Claim 1, in which the content of heavy compounds is greater than or equal to 15% by weight of the liquid phase.

3. Process according to Claim 2, in which the content of heavy compounds is greater than or equal to 20% by weight of the liquid phase.

4. Process according to any one of Claims 1 to 3, in which the heavy compounds other than glycerol have a boiling point under a pressure of 1 bar absolute (100 kPa) which is greater than or equal to 220°C.

5. Process according to Claim 4, in which the heavy compounds other than glycerol have a boiling point under a pressure of 1 bar absolute (100 kPa) which is greater than or equal to 240°C.

6. Process according to Claim 5, in which the heavy compounds other than glycerol have a boiling point under a pressure of 1 bar absolute (100 kPa) which is greater than or equal to 250°C.

7. Process according to any one of Claims 1 to 6, in which the reaction between the glycerol, the glycerol ester or the mixture of them and the chlorinating agent takes place in the liquid phase.

8. Process according to any one of Claims 1 to 7, in which the reaction is carried out continuously.

9. Process according to any one of Claims 1 to 8, in which a fraction of the heavy compounds is separated from the dichloropropanol and recycled in the reaction of the glycerol, the glycerol ester or the mixture of them with the chlorinating agent.

10. Process according to any one of Claims 1 to 9, in which a fraction of the heavy compounds is formed during the chlorination reaction of the glycerol, the glycerol ester or the mixture of them.

11. Process according to any one of Claims 1 to 10, in which a carboxylic acid and/or a carboxylic acid derivative is used as catalyst.

12. Process according to any one of Claims 1 to 11, in which the glycerol, the glycerol ester or the mixture of them is obtained from renewable starting materials.

13. Process according to any one of Claims 1 to 12, followed by manufacture of epichlorohydrin by dehydrochlorination of the dichloropropanol.

14. Process according to Claim 13, in which the epichlorohydrin is used in the manufacture of epoxy resins.

15. Process according to any one of Claims 1 to 18, in which the chlorinating agent is a combination of gaseous hydrogen chloride and of an aqueous hydrogen chloride solution or an aqueous hydrogen chloride solution.
